# EUROPEAN PATENT SPECIFICATION

(11) **EP 4 138 771 B1**
(45) Date of publication and mention of the grant of the patent: **06.03.2024**
(21) Application number: 21716770.9
(22) Date of filing: 12.04.2021
(51) Int. Cl.: A61K 8/44, A23L 33/18, A61K 8/64, A61Q 19/00, C12N 5/00

(54) **COMPOSITIONS COMPRISING CYS-PEPTIDES**
ZUSAMMENSETZUNGEN MIT CYS-PEPTIDEN
COMPOSITIONS COMPRENANT DES PEPTIDES CYS

(30) Priority: 20.04.2020 EP 20170403
(43) Date of publication of application: 01.03.2023
(73) Proprietor: Evonik Operations GmbH, 45128 Essen (DE)
(72) Inventor: SCHILLING, Martin, 53121 Bonn (DE); BENEDIKT, Anne, 60431 Frankfurt (DE); JOST, Christina, 64665 Alsbach-Hähnlein (DE); GOMEZ, Mario, 64297 Darmstadt (DE); KESSLER, Christian, 63741 Aschaffenburg (DE)
(74) Representative: Evonik Patent Association
(86) International application number: PCT/EP2021/059379
(87) International publication number: WO 2021/213822

(56) References cited:
- EP-A1- 1 201 137
- EP-A2- 0 264 953
- WO-A1-2018/162346
- WO-A2-2007/027092

## Description

### FIELD OF THE INVENTION

The present invention relates to compositions that are prepared by mixing at least one oligopeptide, preferably a dipeptide, with one amino acid being cysteine (Cys), and a cysteine source selected from free cysteine and optionally cystine (Cys-Cys) wherein the composition has a pH-value of at least 5, preferably of at least 6. The compositions have the advantage of improved stability/solubility compared to free cysteine/cystine and can be used to provide a source of cysteine/cystine to cells, tissues, organs and whole organisms.

Moreover, the present invention relates to biotechnological production processes. More specifically, the present invention relates to improved culture media for use in biotechnological production processes, processes employing such improved media, and to products obtained from the processes using the improved culture media.

### BACKGROUND OF THE INVENTION

Cysteine is an important amino acid that is used in various applications, from human nutrition, cosmetics to cell and tissue culture processes. It is a building block for proteins and for glutathione, which is an important cellular antioxidant and involved in the regulation of redox homeostasis. Various health benefits have been demonstrated for oral nutritional cysteine/cystine supplementation (reviewed by Plaza et al., Molecules 2018, 23, 575)

Cysteine/cystine is also present in cell culture medium formulations. Although cysteine is not an essential amino acid, limitations in cell culture processes for biologics production can lead to severe loss of viability and productivity. On the other hand, overdosing of cysteine can lead to toxic effects (Ritacco et al., Biotechnol Prog., 2018, Vol. 34, No. 6).

A major problem in the formulation of cysteine in highly concentrated liquid nutrient solutions for various application is that it rapidly oxidizes to cystine in the presence of oxygen and cystine has a low solubility of < 1 mM in a pH range between 3 and 9 (Carta et al., J. Chem. Eng. Data 1996, 41, 414-417).

A solution to this problem is the use of well soluble Cys-dipeptides such as (Ala-Cys)₂ and (Lys-Cys)₂ as a source of cysteine/cystine. Those peptides have found use in cell culture, parenteral nutrition and have been investigated for cosmetic applications (see references below).

However, due to additional synthesis and purification steps required to produce dipeptides, they are significantly more expensive than free cysteine and cystine. Therefor there is still a need for more cost-efficient, highly concentrated cysteine/cystine formulations. Depending on the nature of the other amino acid in the peptide, the solubility increase is sometimes not high enough, which poses further restrictions on formulation possibilities.

EP2561065B1 discloses concentrated feed comprising concentrations of cysteine and tyrosine that support maximal cell growth and/or protein, or viral production while avoiding the problems caused by their limited solubility and stability.

US2013/0130317 A1 describes a method for culturing animal cells having an ability to produce a substance, which comprises culturing the animal cells in a medium supplemented with an oligopeptide having one or more L-cysteines and excluding glutathione.

WO2018/162346 A1 describes cell culture media with dipeptides including Ala-Cys, Pro-Cys and Lys-Cys or in the oxidised cystine form. No free cysteine is present.

For the same reason, Cys-peptides have also been evaluated as alternatives to cysteine in parenteral nutrition (Pollack et al., Zeitschrift für Ernährungswissenschaften 1989, 28: 191-200) and cosmetic applications (Tseng et al., J. Agric. Food Chem. 2015, 63: 6181-6188). Further parenteral or oral nutrient supplements for providing cysteine in the form of Cys-peptides are known, for example in EP0264953 A2, EP1201137 A1 and WO2007/027092 A2.

However, synthetic peptides are significantly more expensive than amino acids and lower cost solutions to stabilize cysteine against precipitation are therefore desirable. Reducing agents can be used to prevent cystine formation from cysteine to a certain extent when oxygen can be physically excluded (reference) but are often not desired. Also, this approach does not work in the presence of high oxygen concentrations and supply, such as an aerated bioreactor environment used for cell culture.

For some Cys-peptides (e.g. (Ala-Cys)₂), solubility is still not high enough to prepare highly concentrated stock or feed solutions. However, in modern cell culture bioprocessing, highly concentrated solutions of amino acids and peptides are important to intensify the processes and to avoid excessive liquid processing (e.g. in perfusion systems), which can reduce flexibility and productivity, complicates downstream processing and comes with a higher environmental burden. Similar problems occur in aqueous food and cosmetic products or in nutrient solutions for parenteral nutrition.

### SUMMARY OF THE INVENTION

The above shortcomings of highly concentrated cysteine containing liquid formulations are addressed by the present invention. The invention is defined by the terms of the appended independent claims. Preferred embodiments of the invention are defined by the dependent claims. Surprisingly it was found, that highly concentrated solutions of cysteine can be stabilized against oxidative precipitation by addition of cysteine containing oligopeptides, dipeptides, and/or their disulfides. Even more surprisingly, respective mixtures even resulted in increased solubility of the respective Cys-oligopeptide in some cases. It was also found that cystine can be solubilized by addition of Cys-peptides in the presence of small amounts of cysteine (or other thiols, not investigated).

Obtained compositions represent stable cysteine forms that can be prepared at significantly lower cost per cysteine-equivalent compared to dipeptides alone and thus enable additional more cost sensitive applications than the current ones. An additional advantage is, that they can be used in applications where a certain amount of free cysteine is required, e.g. when the hydrolysis rate of the oligo- or dipeptide to release cysteine or cystine becomes rate limiting.

The compositions can be prepared by mixing defined molar ratios of Cys-oligopeptide or Cys-dipeptide with cysteine or by mixing defined molar ratios of Cys-dipeptide with cysteine and cystine. The preferred molar ratio of the oligopeptide bound cysteine to the free cysteine is 10 or lower, preferably 4 or lower, more preferably 2 or lower, more preferable 1 or lower, more preferable 0.5 or lower, most preferable 0.2 or lower. The mixtures can be in the form of solids (crystalline powders, agglomerates etc) or aqueous solutions. In the case of aqueous solutions, cysteine is added in a concentration of at least 1 mM, preferable at least 10 mM, more preferable at least 50 mM and most preferable at least 100 mM and the dipeptide is added at the appropriate molar ratio described above.

The compositions according to the present invention can also be a component part of a cosmetic product, a nutritional supplement, a nutrient solution for clinical nutrition, or a cell or tissue culture medium (basal, feed or perfusion medium).

The invention thus relates to compositions prepared by mixing at least one oligopeptide, preferably at least one dipeptide, with one amino acid being cysteine (Cys), and a cysteine source selected from free cysteine and optionally cystine (Cys-Cys). The invention thus relates to a culture medium comprising the composition.

The invention further relates to the use of a culture medium of the invention for culturing cells, preferably plant cells, animal cells or mammalian cells.

Another aspect of the invention relates to a method of manufacturing a cell culture product comprising the steps of (i) providing a cell capable of producing said cell culture product; (ii) contacting said cell with a culture medium according to the invention; and (iii) obtaining said cell culture product from said culture medium or from said cell.

Preferred embodiments of the invention are described in further detail in the following detailed description of the invention.

### BRIEF DESCRIPTION OF THE FIGURES

Figure 1 depicts the formation of precipitates in a 100 mM cysteine solution at pH = 7 at 37 °C over 24 hours in the presence of increasing concentrations of (Lys-Cys)₂.
Figure 2 shows reduction of turbidity of a 50 mM (Ala-Cys)₂ suspension by adding increasing amounts of cysteine.
Figure 3 shows stabilization of a 100 mM cysteine solution against oxidative precipitation by addition of various concentrations of (Lys-Cys)₂.
Figure 4: shows the stabilizing effect against oxidative precipitation provided by addition of various concentrations of Cys-peptides to a 50 mM cysteine solution.

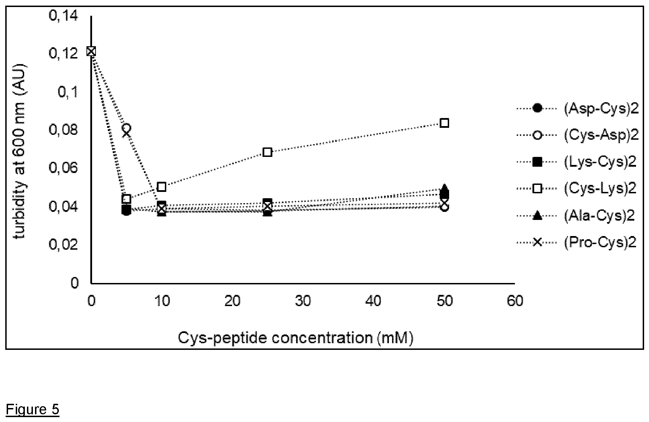

Figure 5 illustrates the stabilizing effect against oxidative precipitation provided by addition of various concentrations of Cys-peptides to a 25 mM cysteine solution.

### DETAILED DESCRIPTION OF THE INVENTION

In the context of the present invention, the expression "natural amino acids" shall be understood to include both the L-form and the D-form of the above listed 20 amino acids. The L-form, however, is preferred. In one embodiment, the term "amino acid" also includes analogues or derivatives of those amino acids.

A "free amino acid", according to the invention, for instance "free" cysteine, is understood as being an amino acid having its amino and its (alpha-) carboxylic functional group in free form, i.e., not covalently bound to other molecules, e.g., an amino acid not forming a peptide bond. Free amino acids may also be present as salts or in hydrate form. When referring to an amino acid as a part of, or in, a dipeptide, this shall be understood as referring to that part of the respective dipeptide structure derived from the respective amino acid, according to the known mechanisms of biochemistry and peptide biosynthesis.

The present invention generally relates to a composition comprising at least one oligopeptide with one amino acid being cysteine (Cys) and (as a cysteine source) free cysteine. Optionally free cysteine and free cystine (Cys-Cys) may be comprised. The terms free cysteine and free cystine (Cys-Cys) shall include the salts or hydrate forms of cysteine and cystine.

Preferably the oligopeptide is a dipeptide.

A "peptide" shall be understood as being a molecule comprising at least two amino acids covalently coupled to each other by alpha-peptide bonds (R¹-CO-NH-R²).

A "polypeptide" shall be understood as being a molecule comprising more than twenty amino acids covalently coupled to each other by peptide bonds (R¹-CO-NH-R²).

An "oligopeptide" shall be understood as being a molecule comprising less than twenty, preferably two to ten amino acids covalently coupled to each other solely by alpha-peptide-bonds (R¹-CO-NH-R²). Glutathione, a Glu-Cys-Gly tripeptide, which contains a gamma-peptide-bond and an alpha-peptide-bond is therefore excluded.

A "dipeptide" shall be understood as being a molecule comprising two amino acids covalently coupled to each other by an alpha-peptide-bond (R¹-CO-NH-R²).

The expression "Xxx", when used herein in connection with an amino acid, shall be understood as referring to any natural amino acid as defined in the following.

An "amino acid", in the context of the present invention, shall be understood as being a molecule comprising an amino functional group (-NH₂) and a carboxylic acid functional group (-COOH), along with a side-chain specific to the respective amino acid. In the context of the present invention, both alpha- and beta-amino acids are included. Preferred amino acids of the invention are alpha-amino acids, in particular the 20 "natural amino" acids including cystine as follows:

| | |
|---|---|
| Alanine | (Ala / A) |
| Arginine | (Arg / R) |
| Asparagine | (Asn / N) |
| Aspartic acid | (Asp / D) |
| Cysteine | (Cys / C) |
| Cystine | (Cyss/C2) |
| Glutamic acid | (Glu / E) |
| Glutamine | (Gln / Q) |
| Glycine | (Gly / G) |
| Histidine | (His / H) |
| Isoleucine | (Ile / I) |
| Leucine | (Leu / L) |
| Lysine | (Lys / K) |
| Methionine | (Met / M) |
| Phenylalanine | (Phe / F) |
| Proline | (Pro / P) |
| Serine | (Ser / S) |
| Threonine | (Thr / T) |
| Tryptophan | (Trp / W) |
| Tyrosine | (Tyr / Y) |
| Valine | (Val/V) |

In the context of the present invention, the expression "natural amino acids" shall be understood to include both the L-form and the D-form of the above listed 20 amino acids. The L-form, however, is preferred. In one embodiment, the term "amino acid" also includes analogues or derivatives of those amino acids.

A "free amino acid", according to the invention (for instance "free cysteine"), is understood as being an amino acid having its amino and its (alpha-) carboxylic functional group in free form, i.e., not covalently bound to other molecules, e.g., an amino acid not forming a peptide bond. Free amino acids may also be present as salts or in hydrate form. When referring to an amino acid as a part of, or in, a dipeptide, this shall be understood as referring to that part of the respective dipeptide structure derived from the respective amino acid, according to the known mechanisms of biochemistry and peptide biosynthesis.

The expression "N-acylated", with reference to a chemical compound, such as an amino acid, shall be understood as meaning that the N-acylated compound is modified by the addition of an acyl group to a nitrogen functional group of said compound. Preferably, the acyl group is added to the alpha-amino group of the amino acid.

In the context of this invention, Cys-peptides forming a disulfide bond via oxidized cysteine residues, shall be described by (Xxx-Cys)₂. The peptides may also be present as salts or in hydrate form. Such disulfide bond mediated dimers of Cys-dipeptides, for instance (Xxx-Cys)₂, are still considered as a dipeptides in the sense of the invention.

Preferably, the composition has a pH-value at 25 °C of at least 5 or preferred of at least 6.

In an advantageous configuration of the present invention, a molar ratio of the peptide-bound cysteine to free cysteine is between 0.1 and 10, preferably between 0.2 and 4 or lower, most preferable between 0.5 and 2 In a preferred embodiment, the oligo- or dipeptides are either in a reduced state (= free thiol) or oxidized state (= disulfide bonded), preferably in an oxidized state In an alternative embodiment, the composition comprises a mixed disulfide of the dipeptide and the cysteine source.

In a preferred embodiment of the present invention, the oligo- or dipeptide further comprises one or more natural amino acids with a solubility of at least > 10 g/l at a pH range between pH 6 and pH 9 and is preferably selected from glycine (Gly), alanine (Ala), serine (Ser), proline (Pro), aspartic acid (Asp), glutamic acid (Glu), lysine (Lys) or arginine (Arg).

It is preferred, when said oligopeptide is a dipeptide which is Xxx-Cys or Cys-Xxx, wherein Xxx is a natural amino acid, preferably the dipeptide preferably being Asp-Cys, Cys-Asp, Lys-Cys, Cys-Lys, Ala-Cys or Pro-Cys. In a preferred configuration, said dipeptide is present in said culture medium at a concentration of at least 1mM, preferably at least 10 mM, more preferably at least 50 mM, more preferably at least 100 mM. At such high concentrations, the composition according to the present invention provides the advantage that the cysteine-containing dipeptides stabilize cysteine against oxidative precipitation.

In preferred embodiments, the oligo- or dipeptide is not N-acylated. N-acylation is known to improve heat stability of certain dipeptide; however, it has been found that N-acylated dipeptides may also lead to inferior viable cell density and viability.

The present invention is also directed to a cosmetic product, a nutritional supplement or nutrient solution for clinical nutrition comprising the composition according to the present invention.

The cosmetic product may be a shampoo, conditioner, lotion, cream or other formulations used to treat skin or hair. Nutritional supplements may be in liquid form, such as syrups or shots, or in solid form, such as capsules, soft-gels, gummies. The compositions can also be part of nutrient solutions for clinical enteral or parenteral nutrition, e.g. part of an amino acid solution such as Aminoven (Fresenius Kabi).

Moreover, the present invention also refers to a cell or tissue culture medium.

Another subject of the present invention is directed to a cell or tissue culture medium comprising the composition according to the present invention, which further comprises at least one carbohydrate, at least one free amino acid, at least one inorganic salt, a buffering agent and/or at least one vitamin. In a particularly preferred embodiment, the culture medium comprises all of at least one carbohydrate, at least one free amino acid, at least one inorganic salt, a buffering agent and at least one vitamin.

In one embodiment of the invention, the culture medium does not contain a growth factor. In accordance with this embodiment, the oligo- or dipeptide of the invention may be used instead of a growth factor for promoting growth and/or proliferation of the cells in culture. In another embodiment of the invention, the culture medium does not contain any lipids.

According to another embodiment of the invention, the culture medium is in liquid form, in form of a gel, a powder, a granulate, a pellet or in form of a tablet.

In preferred embodiments, the culture medium of the invention is a defined medium, or a serum-free medium. For example, the compositions of the invention may be supplemented to the CHOMACS CD medium of Miltenyi Biotech (Bergisch Gladbach, Germany), to the PowerCHO-2 CD medium available from LONZA (Basel, Switzerland), the Acti-CHO P medium of PAA (PAA Laboratories, Pasching, Austria), the Ex-Cell CD CHO medium available from SAFC, the SFM4CHO medium and the CDM4CHO medium of ThermoFisher (Waltham, USA). The dipeptides of the invention may also be supplemented to DMEM medium (Life Technologies Corp., Carlsbad, USA). The invention, however, is not limited to supplementation of the above media.

In other preferred embodiments, the culture medium is a liquid medium in 2-fold, 3-fold, 3.33-fold, 4-fold, 5-fold or 10-fold concentrated form (volume/volume), relative to the concentration of said medium in use. This allows preparation of a "ready-to-use" culture medium by simple dilution of the concentrated medium with the respective volume of sterile water. Such concentrated forms of the medium of the invention may also be used by addition of the same to a culture, e.g., in a fed-batch cultivation or perfusion process.

The cell culture medium (cell or tissue culture basal, feed or perfusion medium) of the present invention may preferably contain all nutrients required for sustained growth and product formation. Recipes for preparing culture media, in particular cell culture media, are well known to the person skilled in the art (see, e.g., Cell Culture Technology for Pharmaceutical and Cell-Based Therapies, Öztürk and Wei-Shou Hu eds., Taylor and Francis Group 2006). Various culture media are commercially available from various sources.

The culture media of the invention may preferably include a carbohydrate source. The main carbohydrate used in cell culture media is glucose, routinely supplemented at 5 to 25 mM. In addition, any hexose, such as galactose, fructose, or mannose or a combination may be used.

The culture medium typically may also include at least the essential amino acids (i.e., His, Ile, Leu, Lys, Met, Phe, Thr, Try, Val) as well as non-essential amino acids. A non-essential amino acid is typically included in the cell culture medium if the cell line is not capable of synthesizing the amino acid or if the cell line cannot produce sufficient quantities of the amino acid to support maximal growth. In addition, mammalian cells can also use glutamine as a major energy source. Glutamine is often included at higher concentrations than other amino acids (2-8 mM). However, as noted above, glutamine can spontaneously break down to form ammonia and certain cell lines produce ammonia faster, which is toxic.

The culture media of the invention may preferably comprise salts. Salts are added to the cell culture medium to maintain isotonic conditions and prevent osmotic imbalances. The osmolality of a culture medium of the invention is about 300 mOsm/kg, although many cell lines can tolerate an approximately 10 percent variation of this value or higher. The osmolality of some insect cell cultures tend to be higher than 300 mOsm/kg, and this may be 0.5 percent, 1 percent, 2 to 5 percent, 5-10 percent, 10-15 percent, 15- 20 percent, 20-25 percent, 25-30 percent higher than 300 mOsm/kg. The most commonly used salts in cell culture medium include Na⁺, K⁺, Mg²⁺, Ca²⁺, Cl⁻, SO₄²⁻, PO₄³⁻, and HCO₃- (e.g., CaCl₂, KCl, NaCl, NaHCO₃, Na₂HPO₄).

Other inorganic elements may be present in the culture medium. They include Mn, Cu, Zn, Mo, Va, Se, Fe, Ca, Mg, Si, and Ni. Many of these elements are involved in enzymatic activity. They may be provided in the form of salts such as CaCl₂, Fe(NO₃)₃, MgCl₂, MgSO₄, MnCl₂, NaCl, NaHCOs, Na₂HPO₄, and ions of the trace elements, such as, selenium, vanadium and zinc. These inorganic salts and trace elements may be obtained commercially, for example from Sigma (Saint Louis, Missouri).

The culture media of the invention preferably comprise vitamins. Vitamins are typically used by cells as cofactors. The vitamin requirements of each cell line vary greatly, although generally extra vitamins are needed if the cell culture medium contains little or no serum or if the cells are grown at high density. Exemplary vitamins preferably present in culture media of the invention include biotin, choline chloride, folic acid, i-inositol, nicotinamide, D-Ca⁺⁺-pantothenate, pyridoxal, riboflavin, thiamine, pyridoxine, niacinamide, A, B₆, B₁₂, C, D₃, E, K, and p-aminobenzoic acid (PABA).

Culture media of the invention may also comprise serum. Serum is the supernatant of clotted blood. Serum components include attachment factors, micronutrients (e.g., trace elements), growth factors (e.g., hormones, proteases), and protective elements (e.g., antitoxins, antioxidants, antiproteases). Serum is available from a variety of animal sources including human, bovine or equine serum. When included in cell culture medium according to the invention, serum is typically added at a concentration of 5-10 %(vol.). Preferred cell culture media are serum-free.

To promote cell growth in the absence or serum or in serum reduced media, one or more of the following polypeptides can be added to a cell culture medium of the invention: for example, fibroblast growth factor (FGF), including acidic FGF and basic FGF, insulin, insulin-like growth factor (IGF), epithelial growth factor (EGF), nerve growth factor (NGF), platelet-derived growth factor (PDGF), and transforming growth factor (TGF), including TGFalpha and TGFbeta, any cytokine, such as interleukins 1, 2, 6, granulocyte stimulating factor, leukocyte inhibitory factor (LIF), etc.

In other embodiments, the cell culture medium does not comprise polypeptides (i.e., peptides with more than 20 amino acids).

One or more lipids can also be added to a cell culture medium of the invention, such as linoleic acid, linolenic acid, arachidonic acid, palmitoleic acid, oleic acid, polyenoic acid, and/or fatty acids of 12, 14, 16, 18, 20, or 24 carbon atoms, each carbon atom branched or unbranched), phospholipids, lecithin (phophatidylcholine), and cholesterol. One or more of these lipids can be included as supplements in serum-free media. Phosphatidic acid and lysophosphatidic acid stimulate the growth of certain anchorage-dependent cells, such as MDCK, mouse epithelial, and other kidney cell lines, while phosphatidylcholine, phosphatidylethanolamine, and phosphatidylinositol stimulate the growth of human fibroblasts in serum-free media. Ethanolamine and cholesterol have also been shown to promote the growth of certain cell lines. In certain embodiment, the cell culture medium does not contain a lipid.

One or more carrier proteins, such as bovine serum albumin (BSA) or transferrin, can also be added to the cell culture medium. Carrier proteins can help in the transport of certain nutrients or trace elements. BSA is typically used as a carrier of lipids, such as linoleic and oleic acids, which are insoluble in aqueous solution. In addition, BSA can also serve as a carrier for certain metals, such as Fe, Cu, and Ni. In protein-free formulations, non-animal derived substitutes for BSA, such as cyclodextrin, can be used as lipid carriers.

One or more attachment proteins, such as fibronectin, laminin, and pronectin, can also be added to a cell culture medium to help promote the attachment of anchorage-dependent cells to a substrate.

The cell culture medium can optionally include one or more buffering agents. Suitable buffering agents include, but are not limited to, N-[2-hydroxyethyl]-piperazine- N'-[2-ethanesulfonic acid] (HEPES), MOPS, MES, phosphate, bicarbonate and other buffering agents suitable for use in cell culture applications. A suitable buffering agent is one that provides buffering capacity without substantial cytotoxicity to the cells cultured. The selection of suitable buffering agents is within the ambit of ordinary skill in the art of cell culture.

Polyanionic or polycationic compounds may be added to the culture medium to prevent the cells from clumping and to promote growth of the cells in suspension.

In a preferred embodiment, the culture medium is in liquid form. The culture medium, however, can also be a solid medium, such as a gel-like medium, e.g. an agar-agar-, carrageen- or gelatinecontaining medium (powders, aggregated powders, instantized powders etc.). Preferably, the culture medium is in sterile form.

The culture medium of the present invention can be in concentrated form. It may be, e.g., in 2- to 100-fold concentrated form, preferably in 2-fold, 3-fold, 3.33-fold, 4-fold, 5-fold, 10-fold, 20-fold, 50-fold or 100-fold (relative to a concentration that supports growth and product formation of the cells). Such concentrated culture media are helpful for preparing the culture medium for use by dilution of the concentrated culture medium with an aqueous solvent, such as water. Such concentrated culture media may be used in batch culture but are also advantageously used in fed-batch or continuous cultures, in which a concentrated nutrient composition is added to an ongoing cultivation of cells, e.g., to replenish nutrients consumed by the cells during culture.

In other embodiments of the invention, the culture medium is in dry form, e.g., in form of a dry powder, or in form of granules, or in form of pellets, or in form of tablets.

The present invention also relates to the use of a culture medium of the invention for culturing cells. Another aspect of the invention relates to the use of a culture medium of the invention for producing a cell culture product.

A preferred embodiment of the invention relates to the use of a culture medium according to the invention for culturing animal cells or plant cells, most preferred mammalian cells. In specific embodiments the cells to be cultured are CHO cells, COS cells, VERO cells, BHK cells, HEK cells, HELA cells, AE-1 cells, insect cells, fibroblast cells, muscle cells, nerve cells, stem cells, skin cells, endothelial cells and hybridoma cells. Preferred cells of the invention are CHO cells and hybridoma cells. Most preferred cells of the invention are CHO cells. Particularly preferred CHO cells of the invention are CHO DG44 and CHO DP12 cells.

Also included in the scope of the present invention is a method of culturing cells, said method comprising contacting said cells with a cell culture medium according to the invention. In one embodiment of the invention, the method of culturing cells comprises contacting the cell with a basal culture medium under conditions supporting the cultivation of the cell and supplementing the basal cell culture medium with a concentrated medium according to the present invention. In preferred embodiments, the basal culture medium is supplemented with the concentrated feed or medium on more than one day.

Another aspect of the invention relates to a method of producing a culture medium according to the invention, wherein said culture medium comprises a composition according to the invention. Methods of producing a culture medium according to the invention comprise at least one step of adding the composition of the invention to the culture medium. Likewise, an aspect of the invention relates to the use of a composition of the invention for producing a cell culture medium.

Another aspect of the invention relates to a method of modifying a culture medium, wherein said modifying of said culture medium comprises addition of the composition of the invention to said culture medium.

Another aspect of the invention relates to a method of producing a liquid culture medium, said method comprising providing solid medium according to the invention, e.g., in form of a dry powder, or in form of granules, or in form of pellets, or in form of tablets; and dissolving said solid culture medium in an aqueous medium, such as water.

Another aspect of the invention relates to the use of a composition according to the invention in a culture medium for culturing cells. Another aspect of the invention relates to the use of a composition according to the invention for cell culture.

The invention also relates to methods of manufacturing a cell culture product comprising the steps of (i) providing a cell capable of producing said cell culture product; (ii) contacting said cell with a culture medium of the invention; and (iii) obtaining said cell culture product from said culture medium or from said cell. Likewise, the present invention relates to the use of a composition according to the invention for manufacturing a cell culture product.

In preferred methods, the cell culture product is a therapeutic protein, a diagnostic protein, a polysaccharide, such as heparin, an antibody, a monoclonal antibody, a growth factor, an interleukin, virus, virus-like particle or an enzyme

Cultivation of cells, according to the invention can be performed in batch culture, in fed-batch culture or in continuous culture.

### EXAMPLES

### Example 1: Stabilization of cysteine against precipitation by addition of Cys-dipeptide (Lys-Cys)₂

Solutions of substances and dilutions thereof were prepared in 100 mM phosphate buffer, pH = 7 in all experiments in all examples. Precipitation could be avoided in a 100 mM solution of cysteine in a 100 mM phosphate buffer at pH = 7 by the addition of suitable concentrations of (Lys-Cys)₂.

Turbidity caused by precipitation was measured photometrically at 600 nm in a microtiter platebased assay. Liquid volume per well was 200 µl and measurements were conducted over 24 hours at 37 °C in a microplate reader (Tecan). The plates were not covered with a lid.

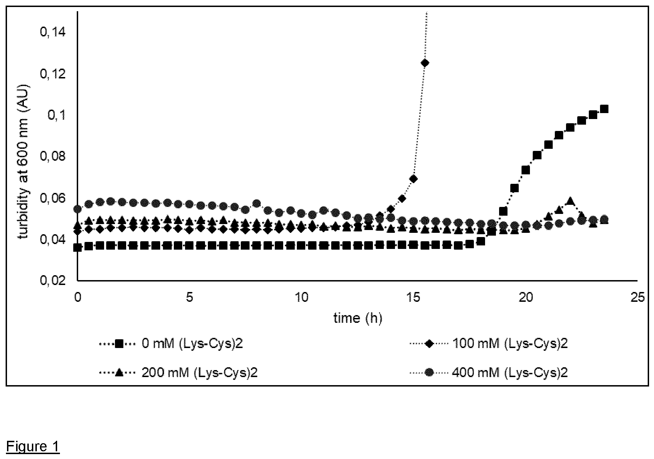

When 100 mM solutions of cysteine were prepared with 0, 100, 200 or 400 mM (Lys-Cys)₂ x 2 HCl at pH = 7, a surprising result was obtained. Precipitation could be seen and measured in the absence of added peptide. However, while 100 mM of (Lys-Cys)₂ even accelerated precipitation, 200 mM and 400 mM (Lys-Cys)₂ effectively prevented precipitation. The precipitates could be observed visually and measured via turbidimetry (see Figure 1).

Figure 1 shows formation of precipitates in a 100 mM cysteine solution at pH = 7 at 37 °C over 24 hours in the presence of increasing concentrations of (Lys-Cys)₂.

### Example 2: Solubilization of cystine by addition of (Lys-Cys)₂ and cysteine

20 ml of the compositions described in Table 1 were prepared in 100 mM phosphate buffer at pH = 7 and incubated in closed 50 ml polypropylene tubes (Falcon) for 24 h. When adding (Lys-Cys)₂ in sufficient concentrations, clear solutions were obtained, whereas the mixtures remained turbid suspensions at lower concentrations.

**Table 1: Millimolar concentrations of mixture components prepared in 100 mM phosphate buffer and incubated for 24 hours at 25 °C with shaking. Visual inspection was carried out the next day.**

| | **Mixture 1** | **Mixture 2** | **Mixture 3** | **Mixture 4** | **Mixture 5** | **Mixture 6** |
|---|---|---|---|---|---|---|
| Cystine | 1.30 | 1.30 | 1.30 | 1.30 | 1.30 | 1.30 |
| (Lys-Cys)₂ | 0.00 | 0,00 | 0.41 | 0.82 | 1.63 | 3.27 |
| Cysteine | 0.00 | 0.13 | 0.13 | 0.13 | 0.13 | 0.13 |
| Visual inspection | suspension | suspension | suspension | suspension | solution | solution |

### Example 3: Solubilization of (Ala-Cys)₂ by addition of cysteine

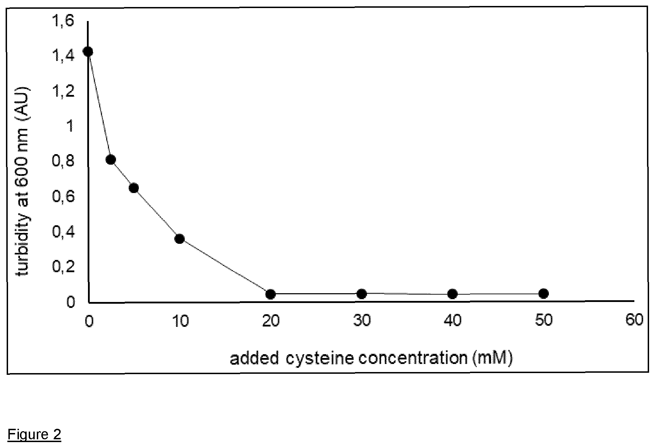

The maximum solubility of (Ala-Cys)z is below 50 mM at pH = 7 and 37 °C. Turbid suspensions of 50 mM (Ala-Cys)₂ in phosphate buffer at pH = 7 were prepared and increasing amounts of cysteine were added. The mixtures were incubated at 37 °C in a microtiter plate (liquid volume per well was 200 µl) and turbidity at 600 nm was measured after 30 minutes. At concentrations of 20 mM of cysteine and higher, (Ala-Cys)z was fully solubilized and no further reduction of turbidity was measured. Solutions were visually transparent (see Figure 2).

Figure 1 shows reduction of turbidity of a 50 mM (Ala-Cys)₂ suspension by adding increasing amounts of cysteine.

### Example 4: Stabilization of a 100 mM cysteine solution against precipitation by addition of Cys-dipeptide (Lys-Cys)₂ under oxidizing conditions

An experiment as described in example 1 was conducted in a microtiter plate. After two hours of incubation at 37 °C, 14 µl of a 5 weight % HzOz solution were added to each well to accelerate oxidation and evaluate stabilization under fully oxidized conditions. Samples containing only cysteine became turbid directly after addition of H₂O₂, which was most likely caused by the formation of cystine For the samples containing (Lys-Cys)₂ no immediate increase in turbidity was observed. Turbidity measurements in the microplate reader were continued subsequently. Results are shown in **Fehler! Verweisquelle konnte nicht gefunden werden.**. Turbidity increase was strongly reduced with increasing concentrations of (Lys-Cys)₂ and no precipitate was observed when 400 mM (Lys-Cys)₂ were added.

Figure 2 shows stabilization of a 100 mM cysteine solution against oxidative precipitation by addition of various concentrations of (Lys-Cys)₂. The mixtures were incubated in a microplate for 2 hours at 37 °C and turbidity was measured photometrically. HzOz was subsequently added to accelerate oxidation and evaluate stability against precipitation under fully oxidized conditions. Turbidity measurement was continued directly after HzOz addition. With cysteine alone, the solution turned directly and visibly turbid, when HzOz was added. With increasing amounts of added (Lys-Cys)₂, the amount of precipitate measured via turbidity was reduced. At 400 mM (Lys-Cys)₂, the mixture remained completely clear during the cause of the measurement.

### Example 5: Stabilization of 25 mM and 50 mM cysteine solutions against precipitation by addition of various Cys-dipeptides under oxidizing conditions

To evaluate if the above described stabilization is also possible by addition of other Cys-dipeptides, experiments as described in example 4 were conducted with the exception that reduced cysteine concentrations of 50 mM and 25 mM were used and that the concentrations of added Cys-dipeptides were 0 (= control), 5, 10, 25 and 50 mM. The effect of (Asp-Cys)₂, (Cys-Asp)₂, (Lys-Cys)₂, (Cys-Lys)₂, (Ala-Cys)₂ and (Pro-Cys)₂ was evaluated. All Cys-peptides were able to prevent precipitation from the cysteine solutions after addition of H₂O₂. Differences in stabilization efficiency were observed between the various Cys-Peptides at cysteine concentrations of 50 mM. The Cys-dipeptides containing lysine residues were the most efficient stabilizers and a molar Cys-peptide to cysteine ratio between 1:2 and 1:5 provided sufficient stability. The results of turbidity measurements with 50 mM cysteine after 4.5 hours are shown in Table 2

**Table 2: Turbidity of a 50 mM cysteine solution mixed with various concentrations of Cys-dipeptides. Turbidity was measured at 600 nm (AU) after 4.5 hours**

| Cys-Peptide concentration | (Asp-Cys)₂ | (Cys-Asp)₂ | (Lys-Cys)₂ | (Cys-Lys)₂ | (Ala-Cys)₂ | (Pro-Cys)₂ |
|---|---|---|---|---|---|---|
| 50 mM | 0.042 | 0.039 | 0.047 | 0.077 | 0.039 | 0.118 |
| 25 mM | 0.039 | 0.279 | 0.045 | 0.067 | 0.037 | 0.762 |
| 10 mM | 1.301 | 1.523 | 0.556 | 0.092 | 1.129 | 1.379 |
| 5 mM | 1.453 | 1.702 | 0.946 | 0.390 | 1.052 | 1.539 |
| 0 mM | 1.279 | 1.279 | 1.279 | 1.279 | 1.279 | 1.279 |

With decreasing concentrations of cysteine, the molar ratio of dipeptide to cysteine required for full stabilization decreased down to the lowest measured ratio of 1:10. This is depicted in Table 3, for the results of turbidity measurements with the 25 mM cysteine solution.

**Table 3: Turbidity of a 25 mM cysteine solution mixed with various concentrations of Cys-dipeptides. Turbidity was measured at 600 nm (AU) after 4.5 hours**

| Cys-Peptide concentration | (Asp-Cys)₂ | (Cys-Asp)₂ | (Lys-Cys)₂ | (Cys-Lys)₂ | (Ala-Cys)₂ | (Pro-Cys)₂ |
|---|---|---|---|---|---|---|
| 50 mM | 0.040 | 0.040 | 0.047 | 0.084 | 0.050 | 0.042 |
| 25 mM | 0.038 | 0.038 | 0.042 | 0.068 | 0.037 | 0.040 |
| 10 mM | 0.039 | 0.037 | 0.041 | 0.050 | 0.037 | 0.039 |
| 5 mM | 0.038 | 0.081 | 0.039 | 0.044 | 0.039 | 0.078 |
| 0 mM | 0.122 | 0.122 | 0.122 | 0.122 | 0.122 | 0.122 |

The results of Table 2 and 3 are visualized in **Fehler! Verweisquelle konnte nicht gefunden werden**. and **Fehler! Verweisquelle konnte nicht gefunden werden.**.

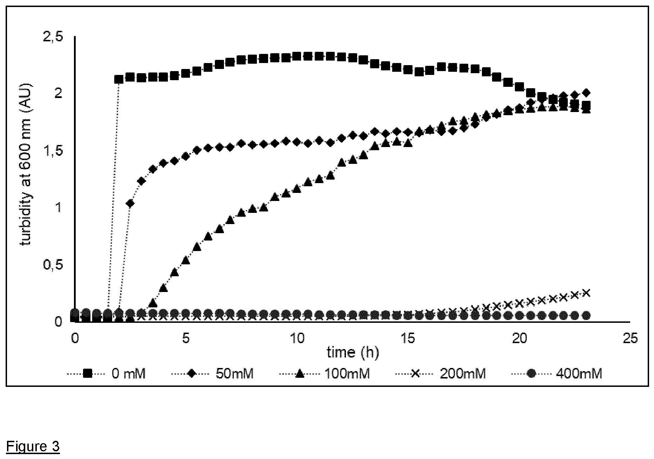

Figure 3 shows stabilizing effect against oxidative precipitation provided by addition of various concentrations of Cys-peptides to a 50 mM cysteine solution.

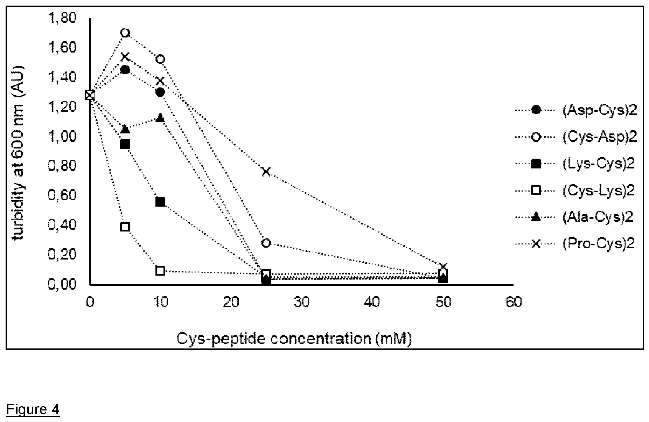

Figure 4 shows stabilizing effect against oxidative precipitation provided by addition of various concentrations of Cys-peptides to a 25 mM cysteine solution.

### Example 6: Stabilization of 50 mM cysteine solutions against precipitation by addition of reduced and oxidized Cys-dipeptides under oxidizing conditions

To evaluate if the above described stabilization is also possible by addition of reduced Cys-dipeptides (= free thiol), the same experimental setup as described in example 4 was chosen. In this case, various concentrations of the reduced dipeptide Cys-Gly and of the oxidized dipeptide (Cys-Gly)₂ were added to a 50 mM cysteine solution. Both Cys-peptides were able to prevent precipitation from the cysteine solutions after addition of H₂O₂. The oxidized dipeptide (Cys-Gly)₂ was more efficient in preventing precipitation as precipitation could already be completely suppressed at a peptide concentration of 25 mM, whereas complete stabilization with the reduced dipeptide Cys-Gly was only obtained at 50 mM. The results are summarized in Table 4.

**Table 4: Turbidity of a 50 mM cysteine solution mixed with various concentrations of reduced Cys-Gly and oxidized (Cys-Gly)z. Turbidity was measured at 600 nm (AU) after 4.5 hours**

| Cys-Peptide concentration | Cys-Gly | (Cys-Gly)₂ |
|---|---|---|
| 50 mM | 0.04 | 0.04 |
| 25 mM | 0.26 | 0.04 |
| 10 mM | 0.37 | 0.07 |
| 5 mM | 0,58 | 1.07 |
| 0 mM | 1.32 | 1.32 |

## Claims

1. A composition, comprising at least one oligopeptide comprising solely alpha-peptide-bonds with one amino acid being cysteine (Cys), and free cysteine.

2. The composition according to claim 1, wherein the oligopeptide is a dipeptide.

3. The composition according to claim 1 or 2, wherein the composition has a pH-value at 25 °C of at least 5, preferably of at least 6.

4. The composition according to any of the preceding claims, wherein a molar ratio of the oligopeptide bound cysteine to the free cysteine is from 0.1 to 10, preferably from 0.2 to 4, most preferable from 0.5 to 2.

5. The composition according to any of the preceding claims, wherein the oligopeptides are either in a reduced state (= free thiol) or oxidized state (= disulfide bonded), preferably in an oxidized state.

6. The composition according to any of the preceding claims, wherein the oligopeptide further comprises one or more natural amino acids with a solubility of at least 10 g/l at 25 °C at a pH range from pH 6 to pH 9, preferably selected from glycine (Gly), alanine (Ala), serine (Ser), proline (Pro), aspartic acid (Asp), glutamic acid (Glu), lysine (Lys) or arginine (Arg), more preferably selected from Ala, Pro, Asp, Lys.

7. The composition according to any of the preceding claims, wherein the oligopeptide is a dipeptide and is Xxx-Cys or Cys-Xxx, wherein Xxx is a natural amino acid, the dipeptide preferably being Asp-Cys, Cys-Asp, Lys-Cys, Cys-Lys, Ala-Cys or Pro-Cys, most preferable Lys-Cys or Cys-Lys.

8. The composition according to any one of the preceding claims, wherein cysteine is added to an aqueous solution at a concentration of at least 1mM, preferably at least 10 mM, more preferably at least 25 mM, most preferably at least 50 mM.

9. A cosmetic product, nutritional supplement, nutrient solution for clinical nutrition, comprising the composition according to any one of the preceding claims.

10. A cell culture medium comprising a composition according to any of claims 1 to 8, said culture medium further comprising at least one carbohydrate, and/or at least one additional free amino acid, and/or at least one inorganic salt, and/or a buffering agent and/or at least one vitamin.

11. The culture medium according to claim 10, wherein said culture medium is in liquid form, in form of a gel, a powder, a granulate, a pellet or in the form of a tablet.

12. The culture medium according to any of claims 10 or 11, wherein said culture medium is in 2- to 100-fold concentrated form, preferably in 2-fold, 3-fold, 3.33-fold, 4-fold, 5-fold or 10-fold concentrated form, relative to the concentration of the culture medium in use.

13. Use of a culture medium according to any one of claims 10 to 12 for culturing cells, preferably as an aqueous stock or feed solution.

14. Use of claim 13, wherein said cells are selected from the list consisting of CHO cells, COS cells, VERO cells, BHK cells, HEK cells, HELA cells, AE-1 cells, insect cells, fibroblast cells, muscle cells, nerve cells, stem cells, skin cells, endothelial cells, immune cells such as NK or T-cells and hybridoma cells.

15. Method of manufacturing a cell culture product comprising the steps of
- providing a cell capable of producing said cell culture product;
- contacting said cell with a culture medium of any one of claims 10 to 12; and
- obtaining said cell culture product from said culture medium or from said cell.

## Patentansprüche

1. Zusammensetzung, umfassend wenigstens ein Oligopeptid, umfassend ausschließlich alpha-Peptid-Bindungen mit einer Aminosäure, die Cystein (Cys) ist, und freies Cystein.

2. Zusammensetzung nach Anspruch 1, wobei das Oligopeptid ein Dipeptid ist.

3. Zusammensetzung nach Anspruch 1 oder 2, wobei die Zusammensetzung einen pH-Wert bei 25 °C von wenigstens 5, vorzugsweise von wenigstens 6, aufweist.

4. Zusammensetzung nach einem der vorangehenden Ansprüche, wobei das Molverhältnis des an das Oligopeptid gebundenen Cysteins zum freien Cystein 0,1 bis 10, vorzugsweise 0,2 bis 4, besonders bevorzugt 0,5 bis 2 beträgt.

5. Zusammensetzung nach einem der vorangehenden Ansprüche, wobei die Oligopeptide entweder in einem reduzierten Zustand (= freies Thiol) oder in einem oxidierten Zustand (= Disulfid-gebunden), vorzugsweise in einem oxidierten Zustand vorliegen.

6. Zusammensetzung nach einem der vorangehenden Ansprüche, wobei das Oligopeptid ferner eine oder mehrere natürliche Aminosäuren mit einer Löslichkeit von wenigstens 10 g/l bei 25 °C und einem pH-Bereich von 6 bis 9 umfasst, die vorzugsweise ausgewählt sind aus Glycin (Gly), Alanin (Ala), Serin (Ser), Prolin (Pro), Asparaginsäure (Asp), Glutaminsäure (Glu), Lysin (Lys) oder Arginin (Arg), mehr bevorzugt aus Ala, Pro, Asp, Lys ausgewählt sind.

7. Zusammensetzung nach einem der vorangehenden Ansprüche, wobei das Oligopeptid ein Dipeptid ist und Xxx-Cys oder Cys-Xxx ist, wobei Xxx eine natürliche Aminosäure ist, das Dipeptid vorzugsweise Asp-Cys, Cys-Asp, Lys-Cys, Cys-Lys, Ala-Cys oder Pro-Cys, am meisten bevorzugt Lys-Cys oder Cys-Lys ist.

8. Zusammensetzung nach einem der vorangehenden Ansprüche, wobei Cystein einer wässrigen Lösung in einer Konzentration von wenigstens 1 mM, vorzugsweise wenigstens 10 mM, mehr bevorzugt wenigstens 25 mM, am meisten bevorzugt wenigstens 50 mM zugesetzt wird.

9. Kosmetisches Produkt, Nahrungsergänzungsmittel, Nährstofflösung zur klinischen Ernährung, umfassend die Zusammensetzung nach einem der vorangehenden Ansprüche.

10. Zellkulturmedium, umfassend eine Zusammensetzung nach einem der Ansprüche 1 bis 8, wobei das Kulturmedium weiterhin wenigstens ein Kohlenhydrat und/oder wenigstens eine zusätzliche freie Aminosäure und/oder wenigstens ein anorganisches Salz und/oder ein Puffermittel und/oder wenigstens ein Vitamin umfasst.

11. Kulturmedium nach Anspruch 10, wobei das Kulturmedium in flüssiger Form, in Form eines Gels, eines Pulvers, eines Granulats, eines Pellets oder in Form einer Tablette vorliegt.

12. Kulturmedium nach einem der Ansprüche 10 oder 11, wobei das Kulturmedium in 2- bis 100-fach konzentrierter Form, vorzugsweise in 2-fach, 3-fach, 3,33-fach, 4-fach, 5-fach oder 10-fach konzentrierter Form, bezogen auf die Konzentration des verwendeten Kulturmediums vorliegt.

13. Verwendung eines Kulturmediums nach einem der Ansprüche 10 bis 12 zur Kultivierung von Zellen, vorzugsweise als wässrige Stamm- oder Zulauflösung.

14. Verwendung nach Anspruch 13, wobei die Zellen aus der Liste ausgewählt sind bestehend aus CHO-Zellen, COS-Zellen, VERO-Zellen, BHK-Zellen, HEK-Zellen, HELA-Zellen, AE-1-Zellen, Insektenzellen, Fibroblastenzellen, Muskelzellen, Nervenzellen, Stammzellen, Hautzellen, Endothelzellen, Immunzellen wie NK- oder T-Zellen und Hybridomzellen.

15. Verfahren zur Herstellung eines Zellkulturprodukts, umfassend die folgenden Schritte
- Bereitstellen einer Zelle, die in der Lage ist, das Zellkulturprodukt zu produzieren;
- Inkontaktbringen der Zelle mit einem Kulturmedium nach einem der Ansprüche 10 bis 12; und
- Gewinnen des Zellkulturprodukts aus dem Kulturmedium oder aus der Zelle.

## Revendications

1. Composition, comprenant au moins un oligopeptide comprenant seulement des liaisons alpha-peptidiques avec un acide aminé qui est la cystéine (Cys), et de la cystéine libre.

2. Composition selon la revendication 1, l'oligopeptide étant un dipeptide.

3. Composition selon la revendication 1 ou 2, la composition ayant une valeur de pH à 25 °C d'au moins 5, préférablement d'au moins 6.

4. Composition selon l'une quelconque des revendications précédentes, le rapport molaire de l'oligopeptide lié à la cystéine sur la cystéine libre étant de 0,1 à 10, préférablement de 0,2 à 4, le plus préférablement de 0,5 à 2.

5. Composition selon l'une quelconque des revendications précédentes, les oligopeptides étant soit dans un état réduit (= thiol libre), soit un état oxydé (= lié par un disulfure), préférablement dans un état oxydé.

6. Composition selon l'une quelconque des revendications précédentes, l'oligopeptide comprenant en outre un ou plusieurs acides aminés naturels dotés d'une solubilité d'au moins 10 g/l à 25 °C à une plage de pH de pH 6 à pH 9, préférablement choisis parmi glycine (Gly), alanine (Ala), sérine (Ser), proline (Pro), acide aspartique (Asp), acide glutamique (Glu), lysine (Lys) ou arginine (Arg), plus préférablement choisis parmi Ala, Pro, Asp, Lys.

7. Composition selon l'une quelconque des revendications précédentes, l'oligopeptide étant un dipeptide et étant Xxx-Cys ou Cys-Xxx, Xxx étant un acide aminé naturel, le dipeptide étant préférablement Asp-Cys, Cys-Asp, Lys-Cys, Cys-Lys, Ala-Cys ou Pro-Cys, le plus préférablement Lys-Cys ou Cys-Lys.

8. Composition selon l'une quelconque des revendications précédentes, de la cystéine étant ajoutée à une solution aqueuse à une concentration d'au moins 1 mM, préférablement d'au moins 10 mM, plus préférablement d'au moins 25 mM, le plus préférablement d'au moins 50 mM.

9. Produit cosmétique, supplément nutritionnel, solution de nutriment pour une nutrition clinique, comprenant la composition selon l'une quelconque des revendications précédentes.

10. Milieu de culture cellulaire comprenant une composition selon l'une quelconque des revendications 1 à 8, ledit milieu de culture comprenant en outre au moins un glucide et/ou au moins un acide aminé libre supplémentaire et/ou au moins un sel inorganique et/ou un agent tampon et/ou au moins une vitamine.

11. Milieu de culture selon la revendication 10, ledit milieu de culture étant source forme liquide, sous forme d'un gel, d'une poudre, d'un granulat, d'une pastille ou sous la forme d'un comprimé.

12. Milieu de culture selon l'une quelconque des revendications 10 ou 11, ledit milieu de culture étant sous forme concentrée 2 à 100 fois, préférablement sous forme concentrée 2 fois, 3 fois, 3,33 fois, 4 fois, 5 fois ou 10 fois, par rapport à la concentration du milieu de culture en utilisation.

13. Utilisation d'un milieu de culture selon l'une quelconque des revendications 10 à 12 pour cultiver des cellules, préférablement en tant qu'une solution aqueuse mère ou d'apport.

14. Utilisation selon la revendication 13, lesdites cellules étant choisies dans la liste constituée par des cellules CHO, des cellules COS, des cellules VERO, des cellules BHK, des cellules HEK, des cellules HELA, des cellules AE-1, des cellules d'insectes, des cellules fibroblastiques, des cellules musculaires, des cellules nerveuses, des cellules souches, des cellules de la peau, des cellules endothéliales, des cellules immunitaires comme des cellules NK ou T et des cellules d'hybridome.

15. Procédé de fabrication d'un produit de culture cellulaire comprenant les étapes de
- fourniture d'une cellule capable de produire ledit produit de culture cellulaire ;
- mise en contact de ladite cellule avec un milieu de culture selon l'une quelconque revendication 10 à 12 ; et
- obtention dudit produit de culture cellulaire à partir dudit milieu de culture ou à partir de ladite cellule.
